# EUROPEAN PATENT APPLICATION

(11) **EP 1 206 932 A1**
(43) Date of publication of application: **22.05.2002**
(21) Application number: 00954920.5
(22) Date of filing: 23.08.2000
(51) Int. Cl.: A61K 7/48

(54) **COSMETICS**

(30) Priority: 24.08.1999 JP 23682699; 21.09.1999 JP 26731799
(71) Applicant: Kao Corporation, Tokyo 103-8210 (JP)
(72) Inventor: IWASE, Norikazu, Kao Corporation Res. Lab., Tokyo 131-8501 (JP); HORI, Kimihiko, Kao Corporation Research Lab., Haga-gun, Tochigi 321-3497 (JP); NONOMURA, Mami, Kao Corporation Research Lab., Haga-gun, Tochigi 321-3497 (JP)
(74) Representative: HOFFMANN - EITLE
(86) International application number: JP0005634
(87) International publication number: WO0113881

(57) **Abstract**

Cosmetics containing one or more components selected from lipids contained in the stratum corneum and analogs thereof, and terpene components other than menthol. The cosmetics are highly efficacious in ameliorating chapped skin and roughened skin and have effects of improving skin qualities such as dry skin or sensitive skin.

## Description

### TECHNICAL FIELD

The present invention relates to cosmetics highly efficacious in ameliorating chapped skin and roughened skin and having effects of improving skin qualities such as dry skin or sensitive skin.

### BACKGROUND ART

Ceramides which are one of intercellular lipids in a stratum corneum are well known to fulfill an important role in the moisturizing function and barrier function of the skin, and it has been reported that the content of ceramides is low in a stratum corneum of a person having dry skin or sensitive skin. Therefore, it has been attempted to apply an external skin care composition containing a natural ceramide, a ceramide analog and/or a phospholipid to such a skin to supply the ceramides decreased in the stratum corneum, thereby improving the function of the skin (Japanese Patent Application Laid-Open Nos. 228048/1987, 216812/1988, 66604/1991, 193754/1991 and 282304/1992, etc.).

In the case of the person having dry skin or sensitive skin, however, such an external skin care composition has involved a problem that its improving effect on chapped skin and roughened skin does not last, and an itch or eczema occurs even for simple irritation because the skin is particularly sensitive, or dermal trouble is easy to occur when nervous stress builds up. Accordingly, there is a demand for development of a cosmetic more effective on dry skin or sensitive skin.

On the other hand, sesquiterpene compounds such as guaiol and cedrol have an inhibitory effect on production of melanine, and external skin care compositions incorporating such a sesquiterpene compound are also known (Japanese Patent Application Laid-Open Nos. 36246/1998 and 36247/1998).

An external skin care composition (Japanese Patent Application Laid-Open No. 128120/1994) incorporating a bioactive substance, a sesquiterpene and a water-soluble polyhydric alcohol is also known. However, the bioactive substance used herein is absorbed in a subcutaneous tissue or migrates into a systemic bloodstream through the skin to act, and does not remain in epidermis, particularly, the stratum corneum to ameliorate chapped skin and roughened skin.

It is an object of the present invention to provide a cosmetic highly efficacious in ameliorating chapped skin and roughened skin even in dry skin or sensitive skin.

### DISCLOSURE OF THE INVENTION

The present inventors have found that when a lipid contained in the stratum corneum or an analog thereof and a terpene component are used in combination, a cosmetic which is greatly improved in effects of ameliorating chapped skin and roughened skin even in dry skin or sensitive skin compared with the single use of these substances, and also excellent in effects of improving skin qualities is provided.

The present invention provides to a cosmetic comprising one or more components selected from lipids contained in the stratum corneum and analogs thereof, and another terpene component than menthol.

The present invention also provides to a cosmetic comprising 0.01 to 60 % by weight of one or more components selected from lipids contained in the stratum corneum and analogs thereof, and 0.05 to 20 % by weight of another terpene component than menthol.

### BRIEF DESCRIPTION OF THE DRAWINGS

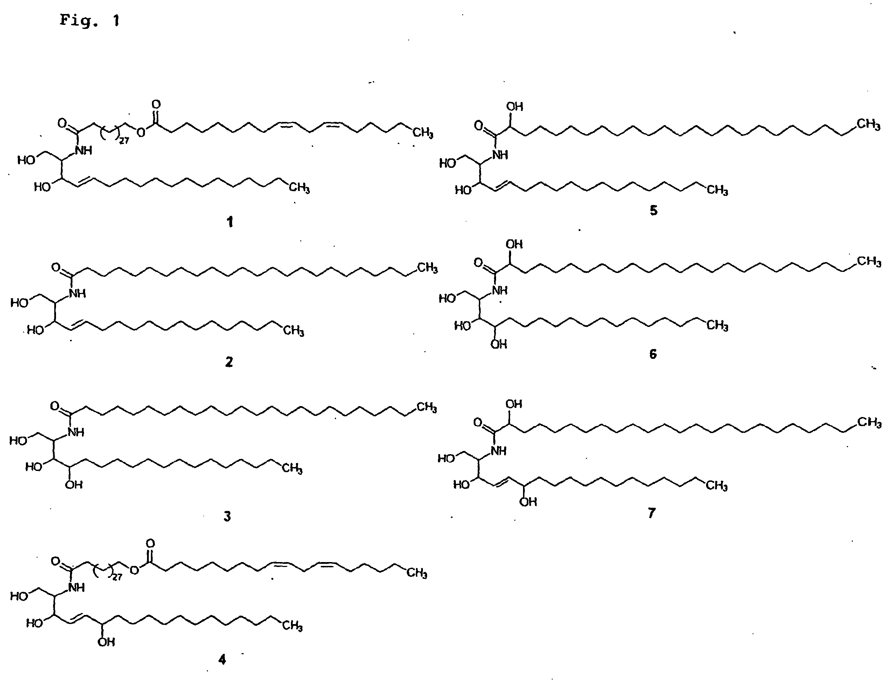

Fig. 1 illustrates examples of the structures of ceramides.

### BEST MODE FOR CARRYING OUT THE INVENTION

Examples of the lipids contained in the stratum corneum used in the present invention include intercellular lipids in the stratum corneum, such as natural ceramides, ceramide analogs, steroids, fatty acid esters of steroids, fatty acids and triglycerides, and besides cerebroside and phospholipids. Among these, natural ceramides, ceramide analogs, phospholipids and fatty acid esters of steroids are preferred.

One or more of known ceramides (ceramides 1 to 7 (Fig. 1) shown on page 2067 in Journal of Lipid Research, Vol. 35, 2060-2068 (1994), etc.) present between cells in the stratum corneum are preferably used as the natural ceramides.

The ceramide analogs include those represented by the following general formulae (1) to (4) and described in Japanese Patent Application Laid-Open No. 216812/1988 (general formula (1)), Japanese Patent Application Laid-Open No. 319263/1996 (general formula (2)), Japanese Patent Application Laid-Open No. 193754/1991 (general formula (3)) and Japanese Patent Application Laid-Open No. 282304/1992
(general formula (4)). 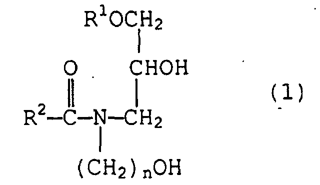 wherein R¹ is a hydrocarbon group having 10 to 26 carbon atoms, R² is a hydrocarbon group having 9 to 25 carbon atoms, and n is a number of 2 to 6.

In the formula, R¹ is preferably a linear or branched alkyl or alkenyl group having 10 to 26 carbon atoms, with an alkyl group having 10 to 18 carbon atoms being particularly preferred. R² is preferably a linear or branched alkyl or alkenyl group having 9 to 25 carbon atoms, with an alkyl group having 9 to 21 carbon atoms being particularly preferred. 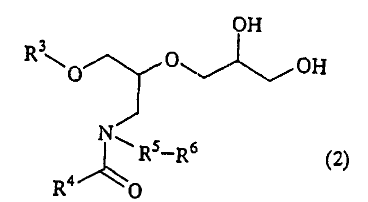 wherein R³ and R⁴ are the same or different from each other and are, independently, a hydrocarbon group having 1 to 40 carbon atoms, which may be hydroxylated, R⁵ is an alkylene group having 1 to 6 carbon atoms or a single bond, and R⁶ is a hydrogen atom, an alkoxy group having 1 to 12 carbon atoms or a 2,3-dihydroxypropyloxy group, with the proviso that R⁶ is a hydrogen atom when R⁵ is a single bond.

In the formula, R³ is preferably a linear or branched alkyl or alkenyl group having 8 to 26 carbon atoms, with an alkyl group having 12 to 22 carbon atoms being particularly preferred. R⁴ is preferably a linear or branched alkyl or alkenyl group having 9 to 25 carbon atoms, with an alkyl group having 11 to 21 carbon atoms being particularly preferred. R⁵ is preferably a linear or branched alkylene group having 1 to 6 carbon atoms, with that having 1 to 3 carbon atoms being particularly preferred. R⁶ is preferably a hydrogen atom, a linear or branched alkoxy group having 1 to 8 carbon atoms or a 2, 3-dihydroxypropyloxy group. 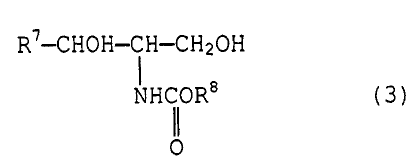 wherein R⁷ is an alkyl or alkenyl group having 11 to 21 carbon atoms, and R⁸ is a hydrocarbon group having 3 to 30 carbon atoms.

In the formula, R⁷ is preferably an alkyl group having 13 to 17 carbon atoms. R⁸ is preferably a linear or branched alkyl or alkenyl group having 8 to 24 carbon atoms. 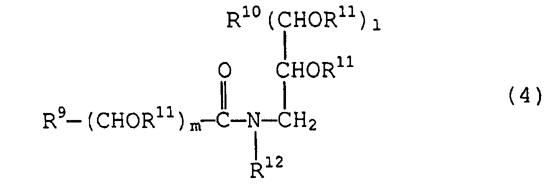 wherein R⁹ is an aliphatic hydrocarbon group having 1 to 49 carbon atoms, which may be hydroxylated, phosphorylated or sulfated, or a sub-substituent group, -(CₐH_{b})-O-Y (Y being a hydrogen atom or a fatty acid residue having 14 to 22 carbon atoms represented by the following formula: 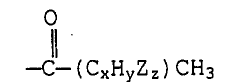 (Z being -OH, -OP₁, -OSO₃⁻ or epoxy oxygen, x being a number of 12 to 20, y being a number of 20 to 40, and z being a number of 0 to 4), a being a number of 7 to 49, and b being a number of 10 to 98); R¹⁰ is an aliphatic hydrocarbon group having 1 to 28 carbon atoms, which may be hydroxylated, phosphorylated or sulfated; R¹¹ is a hydrogen atom, a saccharide residue, a sulfuric acid residue or a phosphoric acid residue P₁ (P₁ being a group: 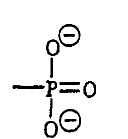 R¹² is a hydrogen atom or a sub-substituent group: 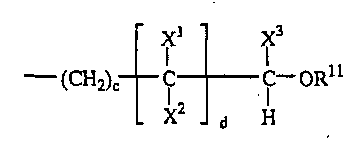 (X¹, X² and X³ being, independently, a hydrogen atom or an alkyl group having 1 to 5 carbon atoms, which may be hydroxylated, c being a number of 0 to 4, d being 0 or 1, and R¹¹ having the same meaning as defined above); and 1 and m are, independently, 0 or 1, with the proviso that 1 + m is 1 or 2 when the number of carbon atoms in R⁹ is 9 to 49.

In the formula, R⁹ is preferably a linear or branched alkyl or alkenyl group having 6 to 32 carbon atoms, with an alkyl group having 10 to 20 carbon atoms being particularly preferred. R¹⁰ is preferably a linear or branched alkyl or alkenyl group having 8 to 22 carbon atoms, with an alkyl group having 10 to 20 carbon atoms being particularly preferred.

R¹¹ is preferably a hydrogen atom, and R¹² is preferably a hydroxyethyl group.

Among these analogs, the ceramide analogs represented by the general formula (1) or (2) are particularly preferred.

The phospholipids include those described in, for example, Japanese Patent Application Laid-Open No. 66604/1991 and represented by the following general formula (5) : 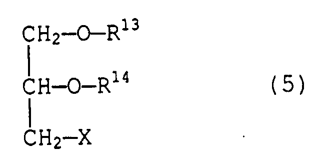 wherein one of R¹³ and R¹⁴ is 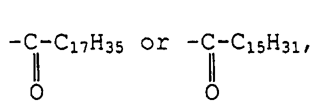 and the other is a hydrogen atom, and X is 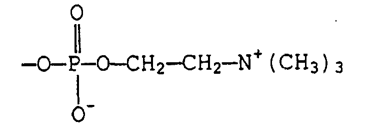 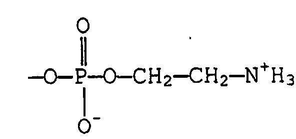 or

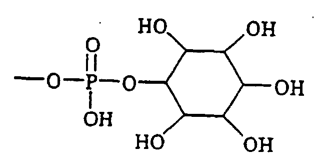

The fatty acid esters of steroids are preferably cholesterol fatty acid esters represented by the following formula (6): 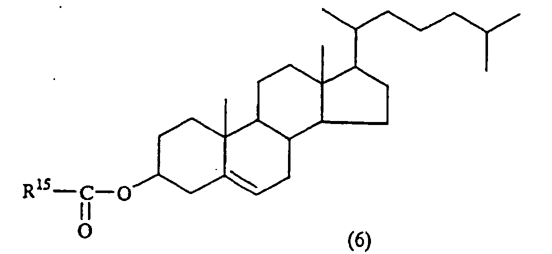 wherein R¹⁵ is a hydrocarbon group having 1 to 25 carbon atoms, which may be hydroxylated.

The lipids contained in the stratum corneum or the analogs thereof may be used alone or in combination and are preferably contained in a proportion of 0.01 to 60 % by weight, particularly 0.05 to 40 % by weight, more preferably 0.1 to 20 % by weight based on the total weight of the cosmetic composition because the resulting cosmetic composition can give users a pleasant feeling upon use.

Examples of the terpene component include terpene hydrocarbons belonging to monoterpene, sesquiterpene or diterpene, terpene alcohols other than menthol, terpene aldehydes, terpene ketones, etc.. Terpene hydrocarbons and terpene alcohols other than menthol are particularly preferred, with sesquiterpene hydrocarbons and sesquiterpene alcohols being more preferred.

The terpene hydrocarbons include α-pinene, β-pinene, camphene, limonene, myrcene, β-caryophyllene, etc.; the terpene alcohols include linalool, geraniol, nerol, citronellol, lavandulol, myrcenol, α-terpineol, borneol, nopol, isobornylcyclohexanol, farnesol, nerolidol, santalol, cedrol, guaiol, vetiverol, patchouli alcohol, etc. Among these, farnesol, santalol, cedrol, guaiol, vetiverol and patchouli alcohol are preferred.

A terpene component which is substantially odorless like cedrol is advantageous in that the degree of freedom of incorporation into a cosmetic composition is high because no influence is imposed on preference for smell.

In the present invention, extracts, steam distilled products or pressed products of plants containing these terpene components may also be used, and such use is preferred.

Examples of such a plant include cedar wood, patchouli, sandal wood, vetiver, ginger root, cimin, thick-haired codium, pepper, rosemary, rose, jasmine, *Valeriana fauriei*, Japanese honeysuckle, thyme, tea and guaiac wood. Cedar wood, patchouli, sandal wood, vetiver, ginger root, pepper, rosemary, rose, jasmine, Japanese honeysuckle and guaiac wood are particularly preferred, with cedar wood, patchouli, sandal wood, vetiver and guaiac wood being more preferred.

These plants may be subjected to extraction, steam distillation, pressing or the like in accordance with a method known *per se* in the art, and the fractions may be further fractionated for use. Essential oils obtained by further purifying these fractions or those derived by a reaction such as acetylation may also be used.

The terpene components may be used alone or in combination, and combined use of at least two of these terpene components or use of a plant extract containing two or more terpene components is particularly preferred because the effects of improving skin qualities are more enhanced.

The terpene component is preferably contained in a proportion of at least 0.05 % by weight, particularly 0.05 to 20 % by weight, more preferably 0.1 to 20 % by weight, still more preferably 1.0 to 10 % by weight based on the total weight of the cosmetic composition from the viewpoint of the effects of ameliorating chapped skin and roughened skin in the dry skin or sensitive skin.

Besides the above-described components, ingredients commonly incorporated in cosmetic compositions, for example, surfactants, oils, amino acids, other moisturizers, powder, ultraviolet light absorbents, gelling agents, anti-inflammatory agents, antioxidants, pH adjusters, menthol and other perfume bases, etc. may be suitably incorporated in the cosmetics according to the present invention.

The cosmetics according to the present invention can be prepared in accordance with a method known *per se* in the art, and may be formulated into any forms such as a solution type, emulsion type, powder-dispersed solution type, powder-dispersed emulsion type and powder-dispersed oil type. The cosmetics according to the present invention are suitable for skin cosmetic compositions such as skin care cosmetic compositions such as toilet lotions, cosmetic emulsions, creams, beauty compositions and cosmetic oils, and makeup cosmetic compositions such as foundations, face powder, lipsticks, cheek rouges, eye shadows and nail enamels. Among others, the cosmetics according to the present invention are particularly preferably provided as skin care cosmetic compositions for ameliorating chapped skin and roughened skin and improving skin qualities such as dry skin or sensitive skin.

### EXAMPLES

### Example 1:

Cosmetic emulsions of their corresponding formulations shown in Tables 1 and 2 were prepared in accordance with a method known *per se* in the art, and evaluated as to the effects of ameliorating chapped skin and roughened skin, and improving skin qualities. The results are shown collectively in Tables 1 and 2.

### (Evaluation methods)

### (1) Degree of amelioration in chapped skin and roughened skin:

Ten women (aged 20 to 30 years) having a chapped skin or roughened skin as panelists were got to use each of the cosmetics twice a day (morning and night) for a week, thereby comparing the conditions of the chapped skin or roughened skin before and after the use of the cosmetic to evaluate it in accordance with the following standard. The results are shown as an average value.
5: Ameliorated more markedly than before use;
4: Ameliorated more considerably than before use;
3: Ameliorated more slightly than before use;
2: Not much more changed than before use;
1: Not more changed at all than before use; and
0: Worsened.

### (2) Degree of improvement in skin qualities:

Ten women (aged 20 to 30 years) as panelists, who had felt their own skin be sensitive, were got to use each of the cosmetics twice a day (morning and night) for a month, thereby comparing the easiness of occurrence of chapped skin or roughened skin in every-day life with before the use of the cosmetic to evaluate it in accordance with the following standard. The results are shown as an average value.
5: Chapped skin or roughened skin was markedly harder to occur than before use;
4: Chapped skin or roughened skin was considerably harder to occur than before use;
3: Chapped skin or roughened skin was slightly harder to occur than before use;
2: Not much more changed than before use, and chapped skin or roughened skin sometimes occurred;
1: Not more changed than before use, and chapped skin or roughened skin often occurred;
0: Chapped skin or roughened skin was easier to occur before the use.

**Table 1**

| Component (% by weight) | Invention product | | | | Comparative product | | |
|---|---|---|---|---|---|---|---|
| | 1 | 2 | 3 | 4 | 1 | 2 | 3 |
| Ceramide analog ^{*1} | 3 | 3 | 3 | 3 | 10 | - | - |
| Patchouli oil ^{*2} | 5 | - | - | 2.5 | - | 5 | - |
| Cedar wood oil ^{*3} | - | 5 | - | 2.5 | - | 5 | - |
| Cedrol | - | - | 5 | - | - | - | - |
| Isostearyl glyceryl ether | 2 | 2 | 2 | 2 | 2 | 2 | 2 |
| Sorbitan monostearate | 2 | 2 | 2 | 2 | 2 | 2 | 2 |
| 2-Octyldodecyl myristate | 10 | 10 | 10 | 10 | 10 | 10 | 10 |
| Squalane | 5 | 5 | 5 | 5 | 5 | 5 | 5 |
| Glycerol | 5 | 5 | 5 | 5 | 5 | 5 | 5 |
| Purified water | Bal. | Bal. | Bal. | Bal. | Bal. | Bal. | Bal. |
| Degree of amelioration in chapped skin and roughened skin | 4.6 | 4.8 | 4.5 | 4.9 | 2.5 | 1.8 | 0.8 |
| Degree of improvement in skin qualities | 4.6 | 4.2 | 4.2 | 5.0 | 1.8 | 2.2 | 1.0 |

| | | | | | | | |
|---|---|---|---|---|---|---|---|
| *1: N-(3-Hexadecyloxy-2-hydroxypropyl)-N-2-hydroxyethyl-hexadecanamide. | | | | | | | |
| *2: Containing 30 % by weight of patchouli alcohol and 20 % by weight of caryophyllene. | | | | | | | |
| *3: Containing 24 % by weight of cedrol. | | | | | | | |

**Table 2**

| Component (% by weight) | Invention product | | | |
|---|---|---|---|---|
| | 5 | 6 | 7 | 8 |
| Ceramide analog *1 | 3 | 3 | 3 | 3 |
| Patchouli oil *2 | 0.5 | - | - | 0.25 |
| Cedar wood oil *3 | - | 0.5 | - | 0.25 |
| Cedrol | - | - | 0.5 | - |
| Isostearyl glyceryl ether | 2 | 2 | 2 | 2 |
| Sorbitan monostearate | 2 | 2 | 2 | 2 |
| 2-Octyldodecyl myristate | 10 | 10 | 10 | 10 |
| Squalane | 5 | 5 | 5 | 5 |
| Glycerol | 5 | 5 | 5 | 5 |
| Purified water | Bal. | Bal. | Bal. | Bal. |
| Degree of amelioration in chapped skin and roughened skin | 4.0 | 4.1 | 4.0 | 4.2 |
| Degree of improvement in skin qualities | 4.0 | 3.6 | 3.6 | 4.2 |

As apparent from Tables 1 and 2, the combined use of a lipid contained in the stratum corneum or an analog thereof and a terpene component permitted extremely markedly ameliorating chapped skin and roughened skin even in dry skin or sensitive skin compared with the single use of these substances.

### Example 2: (Toilet lotion)

A toilet lotion having a composition shown below was prepared in a method known *per se* in the art.

| (Component) | (% by weight) |
|---|---|
| Vetiver oil (containing 60 % by weight of vetiverol) | 2.0 |
| Ceramide analog ^{*1} | 1.0 |
| Polyoxyethylene (20) sorbitan monolaurate | 1.5 |
| Glycerol | 2.0 |
| Paraben | 0.1 |
| Purified water | Balance |

### Example 3: (O/W type cosmetic emulsion)

An O/W type cosmetic emulsion having a composition shown below was prepared in a method known *per se* in the art.

| (Component) | (% by weight) |
|---|---|
| Sandal wood oil (containing 73 % by weight of santalol) | 2.0 |
| Cetyl alcohol | 1.0 |
| Vaseline | 2.0 |
| Squalane | 6.0 |
| Dimethyl polysiloxane | 2.0 |
| Glycerol | 2.0 |
| Ceramide analog ^{*4} | 1.0 |
| Polyoxyethylene (10) monooleate | 1.0 |
| Glycerol monostearate | 1.0 |
| Paraben | 0.2 |
| Purified water | Balance |

| | |
|---|---|
| *4: N-(3-Hexadecyloxy-2-hydroxypropyl)-N-2-hydroxyethylhexadecanamide. | |

### Example 4: (W/O type cream)

A W/O type cream having a composition shown below was prepared in a method known *per se* in the art.

| (Component) | (% by weight) |
|---|---|
| Guaiac wood oil (containing 64 % by weight of guaiol) | 2.0 |
| Dimethyl polysiloxane | 10.0 |
| Methylphenyl polysiloxane | 3.0 |
| Octamethylcyclotetrasiloxane | 12.0 |
| Polyoxyalkylene-modified silicone | 5.0 |
| 1,3-Butylene glycol | 6.0 |
| Ceramide analog ^{*5} | 1.2 |
| Paraben | 0.2 |
| Perfume base | Trace |
| Purified water | Balance |

| | |
|---|---|
| *5: N-[2-(2,3-Dihydroxypropyloxy)-3-hexadecyloxypropyl]-N-3-methoxypropyltetradecanamide. | |

All the cosmetics obtained in Examples 2 to 4 were highly efficacious in ameliorating chapped skin and roughened skin, and had effects of improving skin qualities such as dry skin or sensitive skin.

### INDUSTRIAL APPLICABILITY

The cosmetics according to the present invention are highly efficacious in ameliorating chapped skin and roughened skin and have effects of improving skin qualities such as dry skin or sensitive skin.

## Claims

1. A cosmetic comprising 0.01 to 60 % by weight of one or more components selected from lipids contained in the stratum corneum and analogs thereof, and 0.05 to 20 % by weight of another terpene component than menthol.

2. The cosmetic according to Claim 1, wherein the lipids contained in the stratum corneum and analogs thereof are selected from natural ceramides, ceramide analogs, phospholipids and fatty acid esters of steroids.

3. The cosmetic according to Claim 1 or 2, wherein the lipids contained in the stratum corneum are ceramide analogs or phospholipids represented by the general formulae (1) to (5): 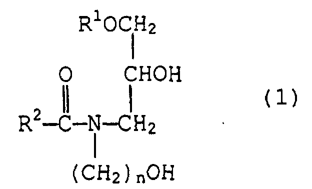 wherein R¹ is a hydrocarbon group having 10 to 26 carbon atoms, R² is a hydrocarbon group having 9 to 25 carbon atoms, and n is a number of 2 to 6; 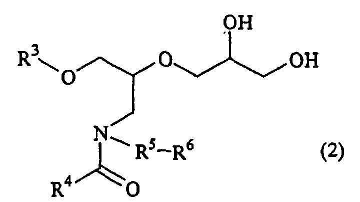 wherein R³ and R⁴ are the same or different from each other and are, independently, a hydrocarbon group having 1 to 40 carbon atoms, which may be hydroxylated, R⁵ is an alkylene group having 1 to 6 carbon atoms or a single bond, and R⁶ is a hydrogen atom, an alkoxy group having 1 to 12 carbon atoms or a 2,3-dihydroxypropyloxy group, with the proviso that R⁶ is a hydrogen atom when R⁵ is a single bond; 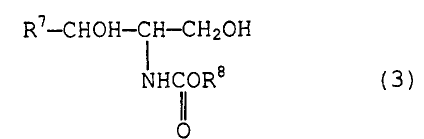 wherein R⁷ is an alkyl or alkenyl group having 11 to 21 carbon atoms, and R⁸ is a hydrocarbon group having 3 to 30 carbon atoms; 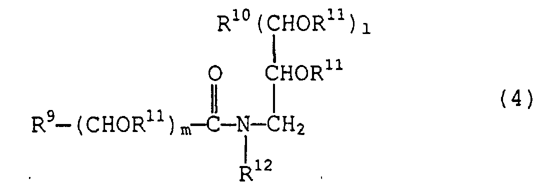 wherein R⁹ is an aliphatic hydrocarbon group having 1 to 49 carbon atoms, which may be hydroxylated, phosphorylated or sulfated, or a sub-substituent group, -(CₐH_{b})-O-Y (Y being a hydrogen atom or a fatty acid residue having 14 to 22 carbon atoms represented by the following formula: 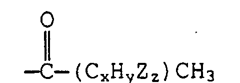 (Z being -OH, -OP₁, -OSO₃⁻ or epoxy oxygen, x being a number of 12 to 20, y being a number of 20 to 40, and z being a number of 0 to 4), a being a number of 7 to 49, and b being a number of 10 to 98); R¹⁰ is an aliphatic hydrocarbon group having 1 to 28 carbon atoms, which may be hydroxylated, phosphorylated or sulfated; R¹¹ is a hydrogen atom, a saccharide residue, a sulfuric acid residue or a phosphoric acid residue P₁ (P₁ being a group: 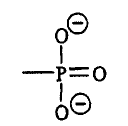 R¹² is a hydrogen atom or a sub-substituent group: 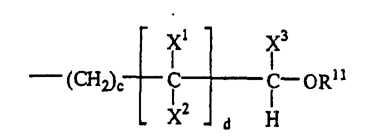 (X¹, X² and X³ being, independently, a hydrogen atom or an alkyl group having 1 to 5 carbon atoms, which may be hydroxylated, c being a number of 0 to 4, d being 0 or 1, and R¹¹ having the same meaning as defined above); and 1 and m are, independently, 0 or 1, with the proviso that 1 + m is 1 or 2 when the number of carbon atoms in R⁹ is 9 to 49; and 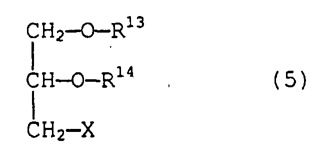 wherein one of R¹³ and R¹⁴ is 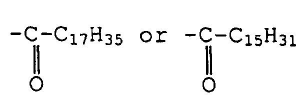 and the other is a hydrogen atom, and X is 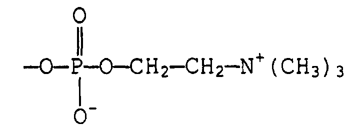 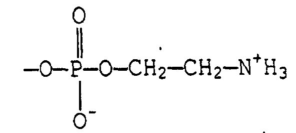 or

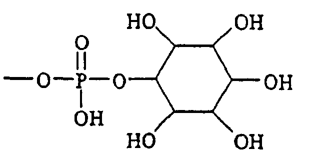

4. The cosmetic according to any one of Claims 1 to 3, wherein the terpene component is terpene hydrocarbon or terpene alcohol.

5. The cosmetic according to any one of Claims 1 to 4, wherein the terpene component is an extract, a steam distilled product or a pressed product of a plant containing terpene hydrocarbon or terpene alcohol.

6. A cosmetic comprising one or more components selected from lipids contained in the stratum corneum and analogs thereof, and cedrol.
